# EUROPÄISCHE PATENTANMELDUNG

(11) **EP 0 706 825 A1**
(43) Veröffentlichungstag der Anmeldung: **17.04.1996**
(21) Anmeldenummer: 95115832.8
(22) Anmeldetag: 07.10.1995
(51) Int. Cl.: B01L 3/14, G06K 19/077, G06K 7/08, A61B 10/00, A61B 19/00

(54) **Probebehälter für Blut, Urin und dergl. mit Datenträger**

(30) Priorität: 10.10.1994 DE 9416270 U
(71) Anmelder: Grieb, Reinhard, D-63633 Birstein (DE)
(72) Erfinder: Grieb, Reinhard, D-63633 Birstein (DE)
(74) Vertreter: Baumann, Eduard, Dipl.-Phys.

(57) **Zusammenfassung**

Die Erfindung bezieht sich auf einen Probenbehälter für Blut, Urin und dergleichen mit einem Datenträger für Personendaten und für Arbeitsdaten.

Zur einfacheren und zuverlässigeren Übertragung und Auswertung sowohl der Personendaten als auch der Arbeitsdaten wird als einziger Datenträger ein Speicherchip (52) innerhalb eines Chipträgers (50) vorgesehen, der über ein Schreib-/Lesegerät (70) Daten von der Tastatur eines Computers einlesen kann. Die Speicherdaten können dann über ein ähnliches Schreib-/Lesegerät vom Laborarzt automatisch ausgelesen werden. Ggf. ist auch eine direkte Verknüpfung mit den Meßwerten möglich. Der Chipträger (50) wird hierbei am Probenbehälter festgeklemmt oder auf andere Weise fest mit diesem verbunden.

## Beschreibung

Die Erfindung bezieht sich auf einen Probebehälter gemäß dem Oberbegriff des Anspruches 1.

Derartige Probenbehälter dienen insbesondere dazu, von Patienten der Ärzte Blut, Urin und dergl. aufzunehmen, zu verschließen und in ein Speziallabor zur Untersuchung zu übersenden. Bisher ist es üblich, sowohl die Personendaten als auch die Informationen über die durchzuführenden Untersuchungen in einem Beiblatt der Probe mitzugeben. Dies ist zeitraubend und umständlich und beinhaltet die Gefahr von Verwechselungen, insbesondere hinsichtlich der Personendaten. Daher geht man in letzter Zeit verstärkt dazu über, die Personendaten über ein sogenanntes Strichcodegerät auf ein entsprechendes Etikett einzulesen und auf den Probenbehälter, in aller Regel Glas, aufzukleben. Beim untersuchenden Laborarzt wird dann das fest mit dem Probenbehälter verbundene Personendaten-Etikett über einen ähnlichen oder gleichen Strichcodeleser in einen Computer eingelesen. Anschließend werden die Arbeitsdaten, d.h. die Informationen über die durchzuführenden Untersuchungen, per Hand von dem nach wie vor erforderlichen beigefügten Datenblatt in den Computer eingelesen. Diese Arbeit ist verhältnismäßig umständlich, darüber hinaus kann es nach wie vor zu Verwechselungen über die durchzuführenden Arbeiten kommen, wenn verschiedene Datenblätter vertauscht werden.

Der Erfindung liegt die Aufgabe zugrunde, einen Probenbehälter gemäß dem Oberbegriff des Anspruches 1 so auszugestalten, daß eine automatische Eingabe der Daten über einen Computer ermöglicht wird, und daß wenigstens Identifizier- Daten stets fest mit dem Probenbehälter verbunden sind.

Die Aufgabe wird erfindungsgemäß durch einen Probenbehälter mit den Merkmalen des Anspruches 1 gelöst. Weitere Ausgestaltungen der Erfindung sind in den Unteransprüchen unter Schutz gestellt.

Beim Erfindungsgegenstand wird als einziger Datenträger ein Speicherchip vorgesehen, der über eine Daten- Übertragungseinrichtung mit Hilfe eines Lesegerätes oder eines Schreib- /Lesegerätes abtastbar ist, das mit einem Computer verbunden ist. Speicherchip und Übertragungseinrichtung sind an einem Chipträger angeordnet, der mit dem Probenbehälter fest oder lösbar verbunden ist. Die Verbindung kann durch ein Paßstück erfolgen, das in ein offenes Ende des Probenbehälters einschiebbar ist, oder über eine Kappe, in welche der Chipträger eingelegt wird, und welche dann über das Ende des Probenbehälters passend und festsitzend aufgeschoben wird.

Als offenes Ende des Probenbehälters dient in aller Regel dasjenige Ende, das der Entnahmenadel für die Probe gegenüber liegt und in welchem der abdichtende Ansaugstempel oder Ansaugkolben für das Einfüllen von Blut oder Urin und dergl. unter Unterdruck angeordnet ist. Das hintere Ende dieses Entnahmestempels kann entfernt oder abgebrochen werden, der Stempel selbst kann unter Umständen bei Bedarf etwas nach innen geschoben werden, damit das Paßstück des Chipträgers in das hintere offene Ende des Probenbehälters eingeschoben werden kann.

Am gleichen Chipträger können mehrere verschiedene Paßstücke für unterschiedliche Innendurchmesser von Probenbehältern vorgesehen werden. Als Speicherchip wird zweckmäßigerweise ein sogenanntes EEPROM eingesetzt, das im Handel frei erhältlich ist und das frei programmierbar ist, d.h. daß Daten beliebig gespeichert und wieder ausgelesen werden können. Zur Übertragung der gespeicherten Daten sowie zum Einlesen der Daten zu Speicherzwecken kann eine spezielle elektromagnetische Spule dienen, die entweder selbst als Antenne fungiert oder mit einer entsprechenden Antenne zusammenwirkt, so daß ein Lesegerät oder ein Schreib- Lesegerät in Form eines induktiven Lesegerätes berührungslos bei einer Entfernung von weniger als etwa 5 cm vom Chipträger die Daten einlesen und auslesen kann.

Als Material für den Chipträger kann verschiedener elektrisch isolierender Kunststoff eingesetzt werden, beispielsweise Epoxid-Harzverguß. Die Kappe kann aus elastischem Kunststoff oder aus Gummi bestehen.

Der Chipträger für den Speicherchip und die mit diesem elektrisch verbundene Daten- Übertragungseinrichtung kann auch in den Betätigungskolben des Probenbehälters integriert werden, beispielsweise in diesem vergossen werden. Hierbei kann der Chipträger auch identisch mit dem Betätigungskolben sein. Verwendet man einen Probenbehälter in Form eines einfachen Reagenzglases mit einem offenen, über einen Verschlußstopfen verschließbaren Ende und einem geschlossenen, häufig abgerundeten Ende, so kann man den Speicherchip nebst Daten- Übertragungseinrichtung auch am Boden dieses Reagenzglases anordnen, entweder gegenüber dem Inhalt abgedichtet oder in einem eingegossenen Chipträger. Weiterhin kann man auch ein einfaches zylindrisches Rohr als Probenbehälter benutzen, in dessen einem offenen Ende der Chipträger mit integriertem, beispielsweise eingegossenem, Speicherchip und Daten-Übertragunseinrichtung abdichtend eingeschoben wird.

Der Speicherchip kann beispielsweise ein EEPROM sein, die Daten-Übertragungseinrichtung eine elektromagnetische Spule, es kann ein induktives reines Lese gerät oder kombiniertes Schreib- /Lesegerät eingesetzt werden. Beim Programmieren oder der Dateneingabe wird an die elektromagnetische Spule des Speicherchips ein entsprechend den Daten moduliertes Wechselstromsignal über eine entsprechende elektromagnetische Sendespule des Lesegerätes oder Schreib- /Lesegerätes gesendet. Umgekehrt wird beim Lesen ein nicht moduliertes Wechselstromsignal gesendet und ein moduliertes Wechselstromsignal empfangen. Das Programmieren bzw. die Dateneingabe und das Lesen efolgen vorzugsweise über eine Computer- Tastatur des Schreib- /Lesegerätes.

Im einzelnen kann ein Schreib- /Lesegerät als Basis einen Mikroprozessor aufweisen, der über einen Treiber betätigt wird und Daten über ein EPROM sowie ein E²PROM bzw. EEPROM empfängt, speichert und abgibt. Weiterhin sind ein Modulator, ein Demodulator und ein Überwachungsglied sowie eine Antenne vorgesehen, desgleichen natürlich eine Energieversorgung.

Die Energieversorgung des Speicherchips erfolgt über das Lesegerät oder das Schreib- /Lesegerät und die gleiche Sendeantenne desselben zur entsprechenden Antenne des Speicherchips in Form der Daten-Übertragunseinrichtung. Beide Antennen können aus induktiven elektromagnetischen Spulen, wie Kupferspulen, bestehen.

Der Speicherchip kann außer einem Gleichrichter, einem Spannungsregler und einem Spannungs- Rücksteller einen Taktgeber, einen Modulator, einen Demodulator, ein EEPROM und einen Kommunikations- Schaltkreis enthalten.

Ausführungsbeispiele der Erfindung sind in der Zeichnung dargestellt.

Es zeigt:
- Fig. 1: einen Probenbehälter und einen noch nicht eingeführten Chipträger,
- Fig. 2: einen anderen kleineren Probenbehälter mit nicht eingeführtem Chipträger,
- Fig. 3: einen weiteren Probenbehälter und eine dahinter angeordnete Kappe, in welche ein Chipträger eingepaßt ist,
- Fig. 4: den Probenbehälter gemäß Figur 1 mit eingesetztem Chipträger,
- Fig. 5: den Probenbehälter gemäß Figur 2 mit eingesetztem Chipträger,
- Fig. 6: den Probenbehälter gemäß Figur 3 mit aufgestülpter Kappe,
- Fig. 7: einen Chipträger in stark vergrößerter Darstellung mit darin eingesetzem Speicherchip, benachbart hierzu angeordneter Daten-Übertragungseinrichtung sowie einem berührungslosen Schreib-/Lesegerät,
- Fig. 8: ein Funktionsschema für die Datenübertragung,
- Fig. 9: einen Probenbehälter, bei dem der Mikrochip und die benachbarte, elektrisch damit verbundene Daten- Übertragungseinrichtung im Betätigungskolben enthalten ist,
- Fig. 10: einen Probenbehälter in Form eines Reagenzglases, an dessen Boden der Chipträger mit Speicherchip und Daten-Übertragungseinrichtung eingegossen ist,
- Fig. 11: einen Probenbehälter in Form eines an beiden Seiten offenen Röhrchens, wobei an einem Ende der Chipträger mit Speicherchip und Daten- Übertragungseinrichtung eingegossen bzw. abdichtend eingeschoben ist,
- Fig. 12: ein Lese- /Schreibgerät zum Lesen, Programmieren und Eingeben von Daten in Form eines Schaltschemas,
- Fig. 13: den Speicherchip mit damit verbundener Daten- Übertragungseinrichtung, der mit dem Probenbehälter verbunden ist, in Form eines Schaltschemas.

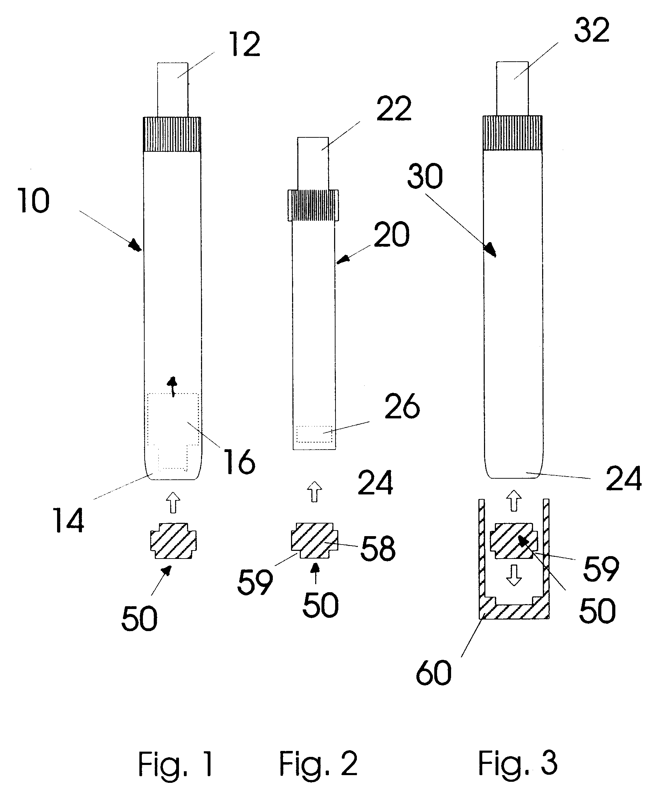

In Figur 1 ist der Probenbehälter, hier in Form eines Kunststoff- oder Glasröhrchens, allgemein mit 10 bezeichnet, an seinem oberen Ende weist er ein Röhrchen 12 auf, auf welches eine sterile Entnahmenadel oder dergleichen aufgesetzt werden kann, welche die Probe, beispielsweise Blut, entweder direkt aus der Vene der Untersuchungsperson aufnimmt, oder welche die Probe aus einem anderen Behälter in den zylindrischen Innenraum des Probenbehälters hineinsaugt. Diesem Zweck dient ein Entnahmekolben 16, der bei seinem Herausziehen nach hinten einen Unterdruck innerhalb des Probenbehälters erzeugt. Hinter dem Probenbehälter 10 ist ein Chipträger 50 angeordnet, der in Pfeilrichtung in das hintere offene Ende 14 des Probenbehälters 10 einschiebbar ist, wobei gleichzeitig der Entnahmekolben oder Entnahmestempel 16, welcher hinten abgebrochen ist oder von welchem der Ziehgriff entfernt wurde, in Pfeilrichtung nach innen eingeschoben wird. Es sei erwähnt, daß die Abdichtung als solche jeweils durch den Entnahmekolben bzw. Entnahmestempel 16 hergestellt wird, so daß es nur darauf ankommt, durch einen entsprechenden Paßsitz eine feste mechanische Klemmverbindung oder andere Verbindung mit dem Probenbehälter 10 herzustellen, damit der Speicherchip 50 stets mit dem Probenbehälter sicher verbunden bleibt.

Figur 2 zeigt ein anderes Ausführungsbeispiel für einen Probenbehälter 20 mit geringerem Durchmesser, vorderem Entnahmeröhrchen 22, hinteren abgebrochenen Entnahmekolben 26 und hinteren offenen Ende 24. Im Vergleich zur Darstellung von Figur 1 ist der Chipträger 50 bei Figur 2 um 180 Grad gedreht, so daß das entgegengesetzte Ende des Chipträgers 50, das als Paßstück 58 mit gegenüber dem Außenumfang des Chipträgers 50 kleinerem Durchmesser ausgebildet ist, dem offenen Ende 24 des Probenbehälters 20 zugewandt ist. Das entgegengesetzte Paßstück 59 mit kleinerem Durchmesser als das erste Paßstück, entsprechend dem Probenbehälter von Figur 1, ist dabei dem Probenbehälter abgewandt.

Figur 3 zeigt ein weiteres Ausführungsbeispiel der Erfindung, bei dem der Chipträger 50 in eine Kappe 60 eingepaßt ist. Wird die Kappe 60 in Pfeilrichtung auf das hintere Ende 24 gegenüber dem Probeentnahmeröhrchen 32 auf den Probenbehälter 30 aufgeschoben, so kann der Chipträger 50 innerhalb der Kappe 60 nach hinten in eine Vertiefung geschoben werden, welche dem hinteren Paßsitz 59 entsprechen kann. Bei diesem Ausführungsbeispiel kann das hintere Ende 24 des Probenbehälters 30 auch geschlossen sein und beispielsweise nur eine einzige vordere Einfüllöffnung im Bereich des Proberöhrchens 32 aufweisen, das auch durch einen einfachen Stöpsel ersetzt sein kann.

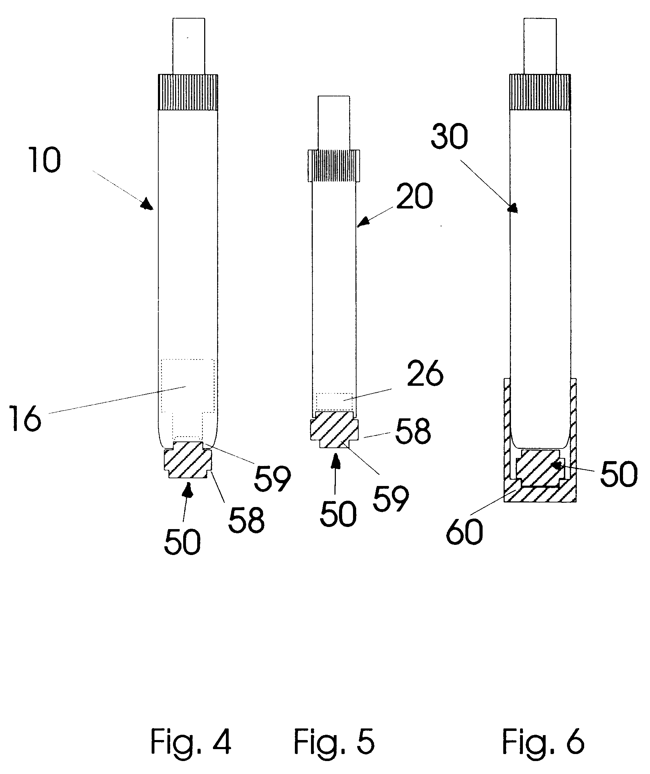

Figur 4 zeigt im Prinzip das Ausführungsbeispiel von Figur 1 mit hinten eingeschobenem Chipträger 50, wobei u.U. das hintere abdichtende Ende des Entnahmekolbens 16 etwas nach innen geschoben werden muß.

Figur 5 zeigt das in Figur 2 dargestellte Ausführungsbeispiel, wobei der Chipträger 50 mit - gegenüber Figur 4 umgedrehtem - Paßstück 58 in das offene hintere Ende des Probenbehälters 20 eingeschoben ist.

Figur 6 zeigt das in Figur 3 dargestellte Ausführungsbeispiel, wobei die Kappe 60 über das hintere Ende des Probenbehälters 30 gestülpt ist, wobei der Chipträger 50 nach hinten bis zum Boden der Kappe 60 geschoben ist.

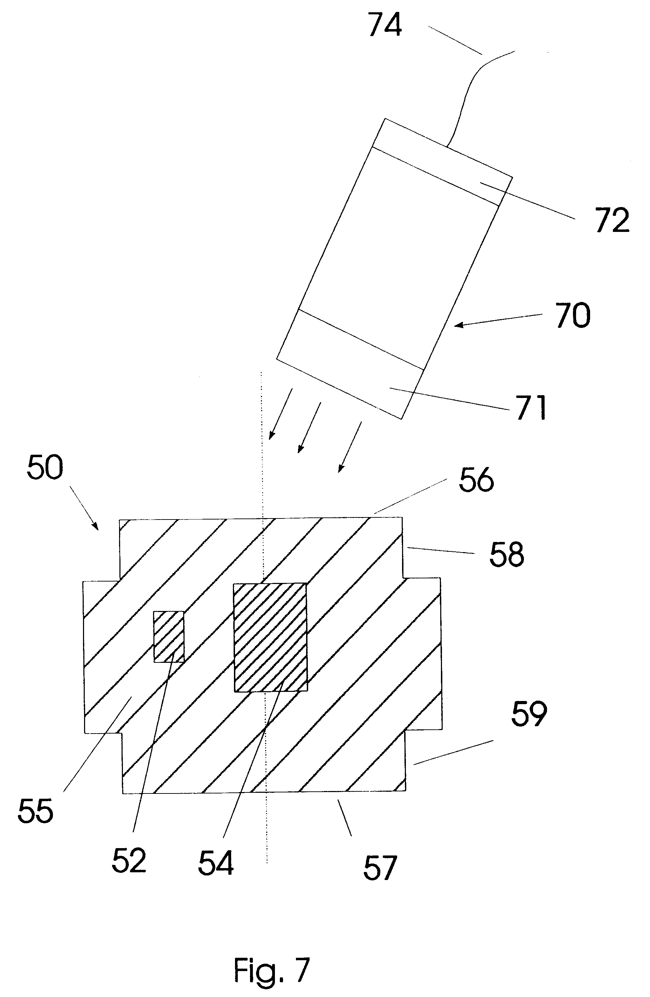

Figur 7 zeigt ein vergrößertes Ausführungsbeispiel des Chipträgers 50, der in Längsschnitt-Darstellung gezeichnet ist. Der Hauptkörper, der beispielsweise aus Kunststoffverguß oder Epoxid-Harzverguß bestehen kann, wird mit 55 bezeichnet. In diesen Verguß ist in einem gewissen Abstand zur Längsachse ein Speicherchip 52 eingezeichnet, der in der Nähe einer in der Achse zentral eingezeichneten Daten-Übertragungseinrichtung 54 in Form einer Spule angeordnet ist.

Oberhalb des Chipträgers 50 rechts ist ein externes Schreib-/Lesegerät 70 angeordnet mit einem vorderen Übertragungsteil 71, das beim berührungslosen Übertragungsvorgang maximal etwa 5 cm vom Chipträger 50 angeordnet sein kann. Das hintere oder interne Übertragungsteil 72 ist über ein Kabel 74 mit einem übliche Computer verbunden, der aufgrund eines speziellen Anwendungsprogrammes vom Speicherchip 52 über die Spule 54 die erhaltenen Daten an den Computer überträgt, wo sie angezeigt und verarbeitet werden können. Umgekehrt können Daten über die Computertastatur in den Computer eingegeben und über das Schreib-/Lesegerät 70 und die Spule 54 innerhalb des Chipträgers 50 an den Speicherchip 52 übertragen werden. Als Speicherchip 52 wird vorteilhaft ein handelsüblicher EEPROM-Chip eingesetzt. In Figur 7 ist im übrigen neu die Vorderfläche des ersten Paßstückes 58 mit 56, die zweite, um 180 Grad versetzte und nach innen kegelförmig abgewinkelte Vorderfläche des entgegengesetzten Paßstückes 59 mit 57 bezeichnet.

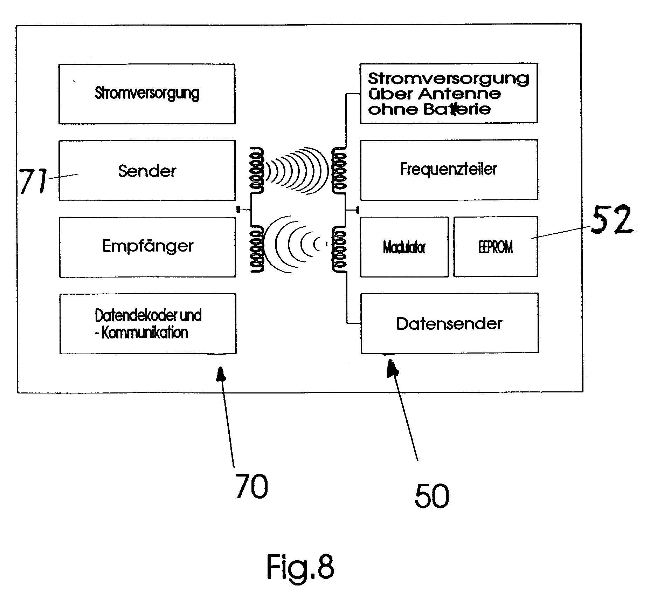

Figur 8 zeigt das Beispiel eines Funktionsschemas für die Datenübertragung zwischen Chipträger 50 bzw. Speicherchip 52 in Form eines EEPROM's und Schreib-/Lesegerät 70 bzw. - nicht dargestelltem - Computer. Der Chipträger 50 bzw. dort angeordnete Speicherchip 52 und die Sende/Empfangs-Spule 54 hat keine eigene Stromversorgung. Diese wird über das externe Schreib-/Lesegerät 70, das mit dem Computer verbunden ist, zugeführt, sie kann beispielsweise etwa 140 KHz, 64 KHz bzw. 128 KHz betragen. Sowohl an der Spule 54 als auch am Sendeteil 71 des Schreib-/Lesegerätes 70 sind jeweils eine Sendeantenne und eine Empfangsantenne angeordnet. Die Datenübertragung erfolgt durch Modulation dieser Sendesfrequenz und bestimmte schaltungsmäßige übliche Maßnahmen, die nicht Gegenstand der Erfindung sind. Erwähnt sei, daß auch eine interne Stromversorgung des Chipträgers mit eigenem Netzanschluß möglich wäre. Die Daten werden über einen Codierer und Decodierer ausgelesen bzw. eingelesen und ausgewertet.

Der erfindungsgemäße Probenbehälter ermöglicht eine feste unverlierbare Anbringung sämtlicher Daten an einem Probenbehälter, die Eingabe über einen üblichen, in jeder Arztpraxis vorhandenen Computer und ein spezielles handliches Schreib-/ Lesegerät und die rasche vollautomatische Auswertung sowohl der Personendaten als auch der Arbeitsdaten beim beauftragten Laborarzt und ggf. das Verknüpfen dieser Daten mit den festgestellten Meßwerten.

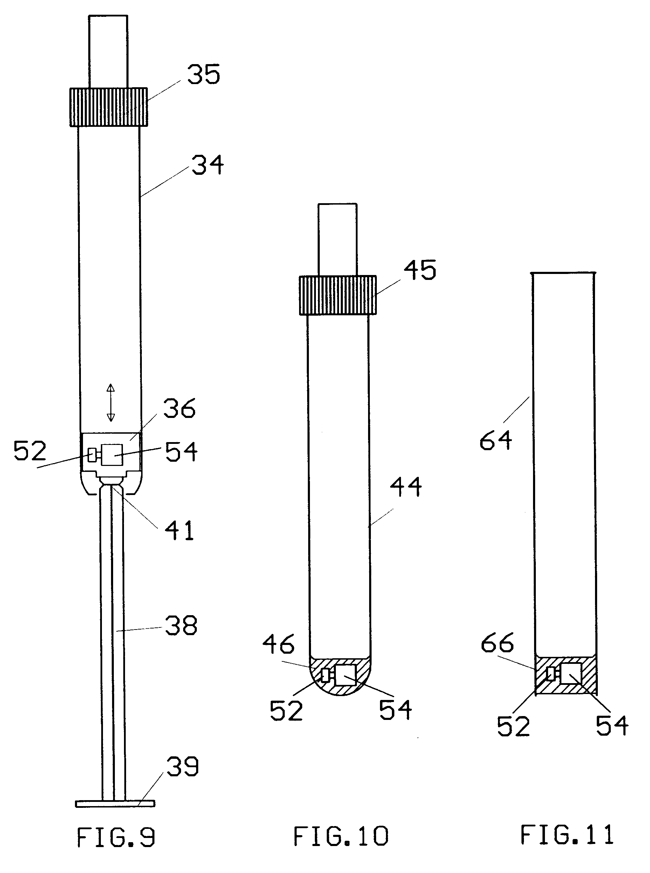

Im Probenbehälter gemäß Fig. 9 ist der Chipträger mit 36 bezeichnet, er stellt gleichzeitig den Betätigungskolben dar, der in Pfeilrichtung innerhalb des Probebehälters 34 aus Glas, Kunststoff oder dergleichen verschiebbar ist, um die Probe zu entnehmen und unter Umständen wieder zu entfernen. In diesem Betätigungskolben 36 ist der Speicherchip 52 sowie die Daten- Übertragungseinrichtung 54 integriert, vorzugsweise abdichtend eingegossen. Nach außen ist der Kolben mit einer Betätigungsstange 38 versehen, an deren Ende der Betätigungshebel 39 angebracht ist Nach dem Befüllen des Probenbehälters kann der Betätigungshebel 38 in bekannter Weise an der Sollbruchstelle 41 für den Versand abgebrochen werden. Die Entnahme der Probe erfolgt dann nach Entfernung des an der gegenüberliegenden Seite des Röhrchens 34 vorgesehenen Deckels 35.

Im Ausführungsbeispiel von Fig. 10 ist als Probenbehälter ein Reagenzglas 44 mit aufgesetztem Deckel 45 vorgesehen. An dessen, üblicherweise abgerundetem, Boden ist der Chipträger 46 eingegossen oder abdichtend eingelassen. Im Chipträger 46 ist der Speicherchip 52 sowie die Daten- Übertragungseinrichtung 54 vorgesehen, vorzugsweise dicht eingegossen. Auch in diesem Falle kann der Chipträger vorzugsweise aus Epoxydharz bestehen.

Fig. 11 zeigt ein an beiden Seiten offenes Probenbehälter- Röhrchen 64, das somit besonders billig ist. An einem Ende ist der Chipträger 66 eingeschoben und mit dem Röhrchen 64 abdichtend verbunden. Im Chipträger 66 ist wiederum der Speicherchip 52 sowie die Daten- Übertragungseinrichtung 54 vorgesehen, welche miteinander elektrisch verbunden sind. Hierbei überträgt die Daten-Übertragungseinrichtung 54 über eine Antenne die Signale zum Lese- /Schreibgerät oder empfängt von diesem Daten.

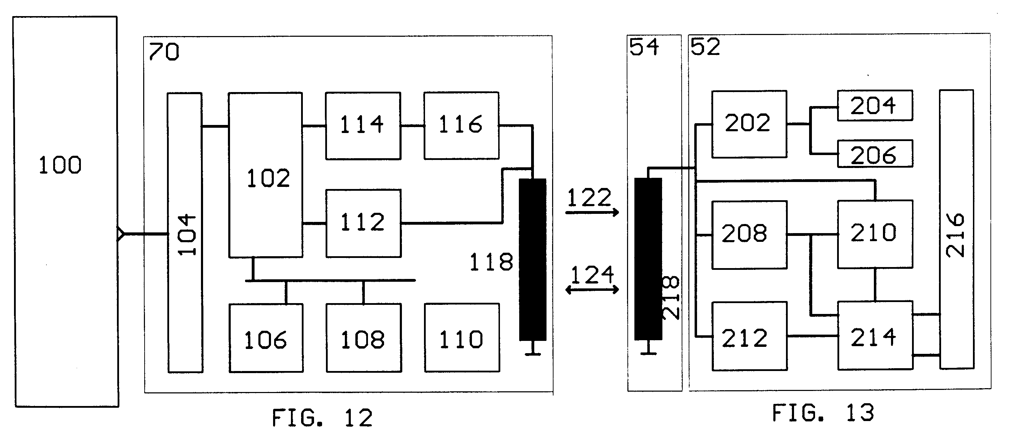

Das Schaltschema von Fig. 12 zeigt ein Lese- /Schreibgerät 70 mit den wesentlichen Untermodulen. Kernstück bildet ein Mikroprozessor 102, der über eine Tastatur 100 und einen Treiber 104 betätigt wird. Vom Mikroprozessor 102 führt ein Leitungsstrang zum EPROM 106 und zum EEPROM 108, eine weitere Leitung führt zu einem Modulator 114, der die Grundfrequenz des Mikroprozessors 102 entsprechend zu sendenden Daten moduliert und über einen RF-Treiber 116 der Sende- /Empfangsantenne 118 zuführt. Andererseits werden von der Antenne 118 empfangene Daten dem Demodulator 112 zugeführt, welcher die Daten zur weiteren Verarbeitung und gegebenenfalls Speicherung an den Mikroprozessor 102 weitergibt.

Zur Schaltungsüberwachung ist ein Überwachungsglied 110 vorgesehen.

Neben das Lese- /Schreibgerät 70 ist die Antenne 218 der Daten-Übertragungseinrichtung 54 angeordnet, die elektrisch mit dem Speicherchip 52 verbunden ist. Eine erste Art von Daten, die vom Lese- /Schreibgerät zum Speicherchip 52 des Probenbehälters übertragen werden, ist mit 122 bezeichnet und dient der Energiezufuhr, eine zweite Art von Daten, die sowohl vom Lese- /Schreibgerät 70 zum Speicherchip 52, als auch in umgekehrter Richtung übertragen werden können, ist mit 124 bezeichnet und dient der Übertragung von Daten zwischen Lese- /Schreibgerät 70 und Speicherchip 52.

Die Antenne 218 der Daten- Übertragungseinrichtung 54 am Probenbehälter, d.h. in der Regel am Chipträger, ist einerseits mit einem Gleichrichter verbunden, dem eine Spannungsregelung 206 sowie eine Rückstellspannung 204 zugeführt wird. Andererseits ist die Antenne 218 mit einem Taktgeber 208, einem Modulator 212 für zu sendende Daten und einem Demodulator 210 für empfangene Daten verbunden. Diese Antenne besteht vorzugsweise aus einer Kupferspule. Kernstück des Speicherchips ist ein EEPROM 216 und ein damit verbundener Kommunikations- Schaltkreis 214.

## Patentansprüche

1. Probenbehälter für Blut, Urin und dergleichen, mit wenigstens einem zugeordneten Datenträger für Personendaten des Probanden, für Arbeitsdaten, d.h. für die mit der Probe durchzuführenden Untersuchungen, sowie zur Identifizierung des Probenbehälters,
dadurch gekennzeichnet,
a) daß der als Datenträger ein Lese- Speicherchip zur Identifizierung des Probenbehälters und/oder für gleichbleibende Untersuchungs- Parameter oder ein Lese- /Schreib- Speicherchip (52) auch zur individuellen Dateneingabe eingesetzt wird,
b) daß eine mit dem Speicherchip (52) elektrisch verbundene und zusammenwirkende Daten- Übertragungseinrichtung (54) vorgesehen ist,
c) die durch ein Lesegerät oder ein Schreib- /Lesegerät (70) abtastbar ist, um sowohl Personendaten und/oder Arbeitsdaten und/oder Identifizierungsdaten im Speicherchip (52) zu speichern, und um umgekehrt die gespeicherten Daten zu lesen,
d) daß der Speicherchip (52) mit dem Probenbehälter fest verbunden ist.

2. Probenbehälter nach Anspruch 1, dadurch gekennzeichnet, daß der Speicherchip (52) und die Daten- Übertragungseinrichtung (54) an einem Chipträger (50) befestigt oder in ihm vergossen sind.

3. Probenbehälter nach Anspruch 2, dadurch gekennzeichnet, daß der Chipträger (50) wenigstens ein Paßstück (58; 59) aufweist, das in ein offenes Ende (14; 24) des Probenbehälters (10; 20) festsitzend einschiebbar ist,
oder daß der Chipträger (50) in eine Kappe (60) paßt, die festsitzend auf ein offenes oder geschlossenes Ende (24) des Probenbehälters (30) aufschiebbar ist.

4. Probenbehälter nach Anspruch 3, dadurch gekennzeichnet, daß der Chipträger (50) verschiedene Paßstücke, vorzugsweise an zwei entgegengesetzten Enden je ein Paßstück (58, 59) mit anderem Durchmesser für Probenbehälter (10, 20) mit verschiedenem Innendurchmesser aufweist.

5. Probenbehälter nach einem der Ansprüche 1 bis 4, dadurch gekennzeichnet, daß der Speicherchip (52) ein EEPROM ist, daß die Daten- Übertragungseinrichtung (54) eine elektromagnetische Spule ist, daß ein induktives Schreib- /Lesegerät (70) eingesetzt wird, welches an die Spule beim Programmieren oder der Dateneingabe ein entsprechend moduliertes Wechselstromsignal sendet, und welches beim Lesen ein nicht moduliertes Wechselstromsignal sendet und ein moduliertes Wechselstromsignal empfängt, wobei Programmieren bzw. Dateneingabe und Lesen vorzugsweise über eine Computer- Tastatur des Schreib- /Lesegerätes (70) erfolgt, und wobei bei der Datenübertragung auch die Energieversorgung des Speicherchips (52) von diesem Gerät über die gleiche Antenne erfolgt.

6. Probenbehälter nach Anspruch 5, dadurch gekennzeichnet, daß die Frequenz des Wechselstromsignales 130 - 160 kHz beträgt.

7. Probenbehälter nach einem der Ansprüche 1 bis 6, dadurch gekennzeichnet, daß der Chipträger (50) ein Kunststoffverguß ist, in welchem der Speicherchip (52) und davon benachbart und elektrisch mit dem Speicherchip verbunden die Daten-Übertragungseinrichtung (54) eingegossen ist.

8. Probenbehälter nach einem der Ansprüche 1 bis 7, dadurch gekennzeichnet, daß die Kappe (60) eine elastische Kunststoff- oder Gummikappe ist.

9. Probenbehälter nach einem der Ansprüche 1 bis 7, dadurch gekennzeichnet, daß der Speicherchip (52) und die mit diesem elektrisch verbundene Daten- Übertragungseinrichtung (54) mit dem Kolben (36) verbunden, vorzugsweise in diesen eingegossen ist.

10. Probenbehälter nach einem der Ansprüche 1 bis 7, dadurch gekennzeichnet, daß der Speicherchip (52) mit der elektrisch mit ihr verbundenen Daten Übertragungseinrichtung (54) an das geschlossene Ende eines U- förmigen Probehälters wie eines Reagenzglases eingedichtet, vorzugsweise über Kunstharz eingegossen ist.

11. Probenbehälter nach einem der Ansprüche 1 bis 7, dadurch gekennzeichnet, daß der Speicherchip (52) sowie die elektrisch mit diesem verbundene Daten- Übertragungseinrichtung (54) an einem Chipträger (66) befestigt sind, der an ein freies Ende eines röhrchenförmigen, zu beiden Seiten offenen Probenbehälters eingeschoben und mit diesem abdichtend verbunden ist.
